(19) Europäisches Patentamt
European Patent Office
Office européen des brevets

(11) Publication number: **0 485 785 A1**

(12) **EUROPEAN PATENT APPLICATION**

(21) Application number: **91118231.9**

(51) Int. Cl.[5]: **C07C 43/303**, C07C 41/58

(22) Date of filing: **25.10.91**

(30) Priority: **13.11.90 JP 306755/90**

(43) Date of publication of application:
**20.05.92 Bulletin 92/21**

(84) Designated Contracting States:
**AT BE DE FR GB NL**

(71) Applicant: **MITSUBISHI RAYON CO., LTD.**
**3-19, Kyobashi 2-chome Chuo-Ku**
**Tokyo 104(JP)**

(72) Inventor: **Miyazaki, Seiji, c/o Central Research Lab.**
**MITSUBISHI RAYON CO. LTD., 20-1, Miyuki-cho**
**Otake-shi, Hiroshima(JP)**
Inventor: **Sonobe, Hiroshi, c/o Central Research Lab.**
**MITSUBISHI RAYON CO. LTD., 20-1, Miyuki-cho**
**Otake-shi, Hiroshima(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **Process and apparatus for producing alpha, beta-unsaturated acetals.**

(57) In a process for preparing $\alpha,\beta$-unsaturated acetals by reacting an $\alpha,\beta$-unsaturated aldehyde with a lower alcohol in the presence of a solid catalyst in a reaction vessel, the improvement comprising introducing a reaction product leaving the reaction vessel into a distillation column at a a stage between the topmost stage to the stage located just above the lowermost stage in the distillation column, and recycling a distillate from the distillation column to the reaction vessel.

EP 0 485 785 A1

## BACKGROUND OF THE INVENTION

### Field of the Invention

The present invention relates to a process and apparatus for the preparation of α,β-unsaturated acetals.

### Discussion of Background

α,β-unsaturated acetals are valuable as perfumes, or as intermediates for the preparation of medical and agricultural chemicals, and as starting materials of resins.

It is known that an acetal can be obtained by reacting an aldehyde with an alcohol under acidic conditions, such as the presence of hydrochloric acid, sulfuric acid, trichloroacetic acid, p-toluene-sulfonic acid, or such solid acid catalysts as strongly acidic ion exchange resin (Japanese Patent Application Laid-open No. 60-188338 and Japanese Patent Publication No. 60-41651). These reactions are equilibrium reactions, and therefore, the conversion of the aldehyde starting material tend to be low. In order to increase the conversion, alcohols are added in an excessive amount, which makes recovery and recycle of unreacted starting materials more difficult.

It is also known that the conversion can be increased by upsetting the equilibrium balance by removal of water formed. Methods for removing water chemically or physically are known, for example by the use of trialkyl orthoformate (Japanese Patent Application Laid-open No. 47-11918). In that process, trialkyl orthoformate is used in an amount equal to the water formed, but the trialkyl orthoformate cannot be recovered. It is also known to use an azeotropic solvent such as hydrocarbons to remove the water (British Patent No. 713,833). However, the hydrocarbon will be distilled off prior to evaporation of water, particularly when methanol is used, so that satisfactory results can not be obtained.

## SUMMARY OF THE INVENTION

The problems outlined above have been solved by the present invention wherein an α,β-unsaturated aldehyde is reacted with a lower alcohol in the presence of a solid catalyst. Unreacted α,β-unsaturated aldehyde and alcohol are then separated by distillation from the resulting α,β-unsaturated acetal and water.

## BRIEF DESCRIPTION OF THE DRAWINGS

Fig. 1 is a first flow diagram of a system for conducting the process according to the present invention.

Fig. 2 is a second flow diagram of a system for conducting the process according to the present invention.

In the drawings, 1 is a distillation pot, 2 is a distillation column, 3 is a condenser, 4 is a liquid distributor, 5 is a reaction vessel, and 6 is a pressure control device by which the pressure in the distillation column can be controlled.

## DETAILED DESCRIPTION OF THE INVENTION

According to the the present invention, α,β-unsaturated acetals represented by the following formula:

$$CH_2 = \overset{\overset{\displaystyle R}{|}}{C}-CH(OR')_2 \qquad (1)$$

wherein R represents a hydrogen atom or a methyl group, and R' represents an alkyl group having 1 to 3 carbon atoms,

are produced by reacting an α,β-unsaturated aldehyde represented by the following formula:

$$CH_2 = \overset{\begin{array}{c} R \\ | \end{array}}{C}-CHO \qquad (2)$$

wherein R represents a hydrogen atom or a methyl group,
with a lower alcohol represented by the following formula:

R'-OH (3)

wherein R' represents an alkyl group having 1 to 3 carbon atoms,
in the presence of a solid catalyst wherein an apparatus comprising a reaction vessel and a distillation column is used.

The catalyst is packed in the reaction vessel.

A reaction product leaving the reaction vessel is introduced into the distillation column at a stage (or plate) between the topmost stage to a stage located just above the lowermost stage of the distillation column. The reaction product contains the acetal and water formed by the reaction as well as unreacted starting materials.

A liquid distillate from the distillation column is recycled to the reaction vessel.

In the present invention, an $\alpha,\beta$-unsaturated acetal can be synthesized economically at a conversion of almost 100%, without the use of a chemical dehydrator or an azeotropic solvent.

In the present invention, since unreacted starting materials are evaporated by distillation and reintroduced into the reaction vessel again, conditions which do not allow for distillation of water as a by-product from the distillation column should be selected. Therefore, the starting $\alpha,\beta$-unsaturated aldehyde and lower alcohol must have boiling points lower than the boiling point of water. Suitable $\alpha,\beta$-unsaturated aldehyde are acrolein and methacrolein. Suitable alcohols include, methanol and ethanol.

The quantities of $\alpha,\beta$-unsaturated aldehyde and lower alcohol can be selected for optimal conditions, but preferably, the lower alcohol is used in an amount of 2 to 4 moles per mole of the $\alpha,\beta$-unsaturated aldehyde. In order to repress the polymerization of the $\alpha,\beta$-unsaturated aldehyde, a polymerization inhibitor is preferably added to the liquid mixture of starting materials.

Either a fixed bed type reactor or a fluidized bed type reactor can be used as the reaction vessel. The temperature of the reaction vessel is not particularly restricted, and the optimal temperature is lower than the boiling point of the reaction product. Nevertheless, since the reaction of the present invention is an exothermic, the reaction vessel should preferably be cooled to remove heat. A solid catalyst capable of advancing the acetalization reaction is packed in the reaction vessel. Strong acidic ion exchange resins, zeolite or active aluminosilicate are suitable catalysts.

The reaction product leaving the reaction vessel is introduced between any stage from the topmost stage to the stage located just above the lowermost stage in the distillation column, wherein the acetal and water is separated from unreacted $\alpha,\beta$-unsaturated aldehyde and lower alcohol. The actual point of introduction into the column is determined according to the physical properties of the reaction product, but if the reaction product is directly introduced into the lowermost stage of the distillation column or into the distillation pot, water will tend to be distilled with the unreacted starting materials.

As the reaction advances, $\alpha,\beta$-unsaturated acetal and water are accumulated in the distillation pot. Since $\alpha,\beta$-unsaturated acetals will decompose in the presence of water under acidic conditions, a base or buffer is preferably added to the distillation pot such as a carboxylic acid salt, a carbonic acid salt, a bicarbonic acid salt, a hydrogenphosphoric acid salt, a dihydrogenphosphoric acid salt, a hydroxides or alkoxide of alkali metal, alkaline earth metal, Zn or Cd, any of which may be used in solid form or solution. The pH of the reaction product in the distillation pot is adjusted to 6 to 8. If the pH value is adjusted to a highly alkaline side, the acetal can be decomposed as under acidic conditions, and good results would not be obtained.

According to the present invention, an $\alpha,\beta$-unsaturated acetal of high concentration can be economically produced at a high conversion of almost 100%, without using a chemical dehydrator or an azeotropic solvent.

## EXAMPLES

Now, the present invention will be described in further detail with reference to Examples. However, it should be understood that the present invention is by no means restricted by such specific Examples.

Example 1

A 3-liter flask distillation pot (1) was charged with 1282 g (40 moles) of methanol, 560 g (10 moles) of acrolein, 1 g of hydroquinone and 2 g of sodium hydrogencarbonate, and a distillation column (2) and a reaction vessel (5) provided with a cooling device were attached. The reaction vessel (5) was packed with 50 mℓ of a strongly acidic ion exchange resin (Amberlist 15 supplied by Rohm and Haas) as the solid catalyst. The apparatus was assembled so that the reaction product leaving the reaction vessel (5) was returned to the topmost stage of the distillation column (2). The apparatus was provided also with a condenser (3), a liquid distributor (4), and a pressure control device (6) as shown in Fig. 1. Then, the distillation pot (1) was heated and reaction was carried out for 12 hours under the condition of 700 torr while maintaining the temperature within the reaction vessel (5) at a level lower than 20°C. After the end of the reaction, the content in the distillation pot (1) was a 54% acetal solution containing 1001 g of acrolein dimethylacetal. The conversion of acrolein in this reaction was 98%.

Comparative Example 1

A 2-liter flask was charged with 513 g (16 moles) of methanol, 225 g (4 moles) of acrolein and 1 g of hydroquinone under ice cooling, and 163 g of a strongly acidic ion exchange resin (Amberlist 15) was added to the flask to effect reaction. After the reaction for 12 hours, the catalyst was removed by filtration, and a 29% solution of acrolein dimethylacetal was obtained. The conversion of acrolein in this reaction was 53%.

Example 2

A 3-liter flask distillation pot (1) was charged with 1154 g (36 moles) of methanol, 842 g (12 moles) of methacrolein, 2 g of hydroquinone and 2 g of sodium carbonate, and a distillation column (2) and a reaction vessel (5) provided with a cooling device were attached. The reaction vessel (5) was packed with 50 mℓ of a strongly acidic ion exchange resin (Amberlist 15) as the solid catalyst. The apparatus was assembled so that the reaction product leaving the reaction vessel (5) was returned to the middle stage of the distillation column (2). The apparatus was provided also with a condenser (3) and a liquid distributor (4) as shown in Fig. 2. Then, the distillation pot (1) was heated and reaction was carried out for 12 hours while maintaining the temperature within the reaction vessel (5) at a level lower than 20°C. After the end of the reaction, the reaction liquid in the distillation pot (1) was separated into two layers. The upper layer was composed of an 82% acetal solution containing 1192 g of methacrolein dimethylacetal. The conversion of methacrolein in this reaction was 99%.

Comparative Example 2

A 2-liter flask was charged with 514 g (16 moles) of methanol, 309 g (4 moles) of methacrolein and 1 g of hydroquinone under ice cooling, and 233 g of a strongly acidic ion exchange resin (Amberlist 15) was added as the catalyst into the flask to effect a reaction. After the reaction for 12 hours, the catalyst was removed by filtration to obtain a 23% solution of methacrolein dimethylacetal. The conversion of methacrolein in this reaction was 41%.

## Claims

**1.** In a process for preparing $\alpha,\beta$-unsaturated acetals represented by the following formula:

$$CH_2 = \overset{\displaystyle R \atop |}{C}-CH(OR')_2 \qquad (1)$$

wherein R represents a hydrogen atom or a methyl group, and R' represents an alkyl group having 1 to 3 carbon atoms,
wherein an $\alpha,\beta$-unsaturated aldehyde represented by the following formula:

$$CH_2 = \overset{\displaystyle R}{\overset{|}{C}}-CHO \qquad (2)$$

wherein R represents a hydrogen atom or a methyl group,
is reacted with a lower alcohol represented by the following formula:

R'-OH (3)

wherein R' represents an alkyl group having 1 to 3 carbon atoms,
by being passed over a solid catalyst in a reaction vessel, the improvement comprising introducing the reaction liquid leaving the reaction vessel into a distillation column at a stage between the topmost stage to the stage located just above the lowermost stage in the distillation column, and recycling a distillate from the distillation column back to the reaction vessel.

**2.** The process according to claim 1, wherein said reaction vessel is provided with a cooling device.

**3.** The process according to claim 1 or 2, wherein at least one member selected from the group consisting of a carboxylic acid salt, carbonic acid salt, bicarbonic acid salt, hydrogenphosphoric acid salt, dihydrogenphosphoric acid salt, hydroxide or alkoxide of alkali metal, alkaline earth metal, Zn and Cd is added in solid form or solution into a pot of the distillation column to adjust the liquid in the pot to pH 6 to 8.

**4.** The process according to claim 1, 2 or 3, wherein the starting materials are first introduced into the pot of the distillation column to initiate the reaction.

**5.** The process according to claim 1, wherein the starting materials are introduced directly into the reaction vessel to initiate the reaction.

**6.** A process for preparing $\alpha,\beta$-unsaturated acetals represented by the following formula:

$$CH_2 = \overset{\displaystyle R}{\overset{|}{C}}-CH(OR')_2 \qquad (1)$$

wherein R represents a hydrogen atom or a methyl group, and R' represents an alkyl group having 1 to 3 carbon atoms,
by reacting an $\alpha,\beta$-unsaturated aldehyde represented by the following formula:

$$CH_2 = \overset{\displaystyle R}{\overset{|}{C}}-CHO \qquad (2)$$

wherein R represents a hydrogen atom or a methyl group,
with a lower alcohol represented by the following formula:

R'-OH (3)

wherein R' represents an alkyl group having 1 to 3 carbon atoms,
in a reaction vessel in the presence of a solid catalyst, separating an unreacted $\alpha,\beta$-unsaturated aldehyde and alcohol from resulting $\alpha,\beta$-unsaturated acetal and water by distillation, and therefore recycling the unreacted $\alpha,\beta$-unsaturated aldehyde to the reaction vessel.

7. The process according to claim 6, wherein the reaction is carried out at a temperature lower than the boiling temperature of a reaction product.

8. The process according to claim 7, wherein the temperature is lower than about 20 °C.

9. The process according to claim 6, wherein at least one member selected from the group consisting of a carboxylic acid salt, carbonic acid salt, bicarbonic acid salt, hydrogenphosphoric acid salt, dihydrogenphosphoric acid salt, hydroxide or alkoxide of alkali metal, alkaline earth metal, Zn and Cd is added in solid form or solution into the pot of the distillation column.

10. The process according to claim 9, wherein the liquid in the pot of the distillation column is adjusted to pH 6 to 8.

11. An apparatus for producing $\alpha,\beta$-unsaturated acetal comprising a distillation column, distillation pot connected with the distillation column, condenser for a distillate from the distillation column, liquid distributor for the condensate, and a reactor, inlet of the reactor being connected with the distributor through a conduit and outlet of the reactor being connected with the distillation column through another conduit.

12. An apparatus for producing $\alpha,\beta$-unsaturated acetal according to claim 11, wherein the distillation column is connected with the distillation pot through a conduit.

13. An apparatus for producing $\alpha,\beta$-unsaturated acetal according to claim 11, wherein the distillation column and the distillation pot are integrally constructed.

14. An apparatus for producing $\alpha,\beta$-unsaturated acetal according to claim 11, wherein a pressure control device is further provided, the pressure control device being directly connected with a conduit diverging from the conduit connecting the condenser with the liquid distributor.

Fig. 1

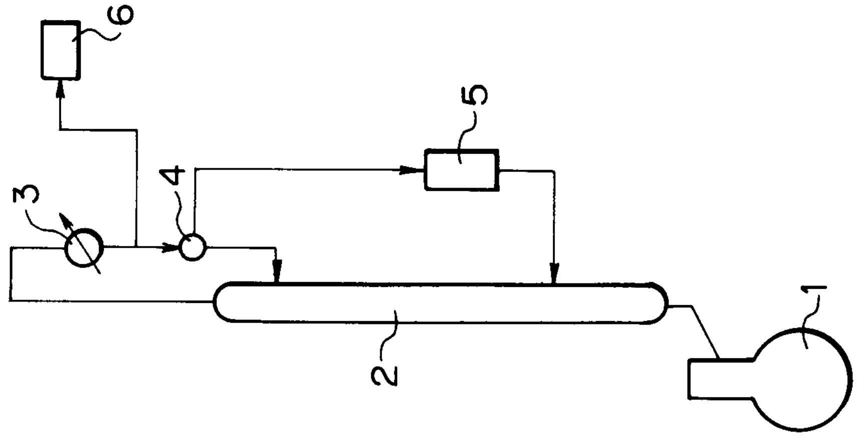


Fig. 2

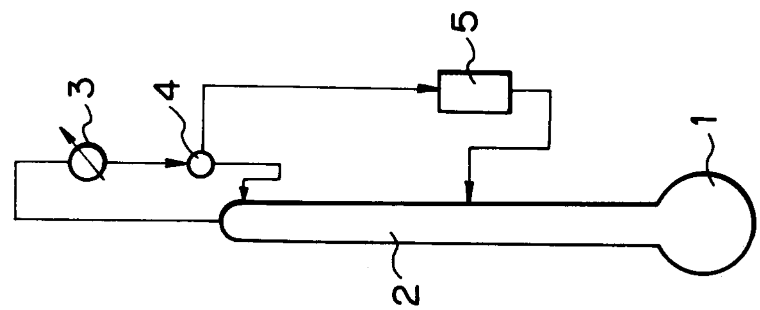

European Patent Office

# EUROPEAN SEARCH REPORT

Application Number

EP 91 11 8231

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 150 280 (DEGUSSA)<br>* examples 1,3,4 *<br>--- | 1-14 | C07C43/303<br>C07C41/58 |
| A | DE-A-2 929 827 (BASF)<br>* claims; example; figure *<br>--- | 1-14 | |
| A | US-A-2 626 283 (R. R. WHETSTONE)<br>* example *<br>----- | 1-14 | |
| | | | TECHNICAL FIELDS SEARCHED (Int. Cl.5 ) |
| | | | C07C |

The present search report has been drawn up for all claims

| Place of search | Date of completion of the search | Examiner |
|---|---|---|
| THE HAGUE | 11 MARCH 1992 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)